# EUROPEAN PATENT SPECIFICATION

(11) **EP 3 061 062 B1**
(45) Date of publication and mention of the grant of the patent: **16.11.2022**
(21) Application number: 14856077.4
(22) Date of filing: 24.10.2014
(51) Int. Cl.: G16H 10/40, G16H 10/60, G16H 15/00, G01N 33/50

(54) **SYSTEMS AND METHODS FOR ORDERING LABORATORY TESTS AND PROVIDING RESULTS THEREOF**
SYSTEME UND VERFAHREN ZUR ANFORDERUNG VON LABORTESTS UND ZUR DARSTELLUNG DER ERGEBNISSE DAVON
SYSTÈMES ET PROCÉDÉS DE COMMANDE DE TESTS DE LABORATOIRE ET DE FOURNITURE DES RÉSULTATS DE CEUX-CI

(30) Priority: 24.10.2013 US 201361895239 P
(43) Date of publication of application: 31.08.2016
(73) Proprietor: Labrador Diagnostics LLC, Wilmington, Delaware 19801 (US)
(72) Inventor: BALWANI, Sunny, Palo Alto, California 94304 (US)
(74) Representative: Avidity IP
(86) International application number: PCT/US2014/062266
(87) International publication number: WO 2015/061743

(56) References cited:
- WO-A2-2005/109238
- US-A1- 2002 161 606
- US-A1- 2003 217 037
- US-A1- 2005 075 543
- US-A1- 2005 075 543
- US-A1- 2011 295 517
- US-A1- 2013 109 106
- US-B1- 8 333 717

## Description

### BACKGROUND

Identification of a person's physiological status is often a first step in improving the person's well-being. For instance, if a person is unaware that he or she has high blood pressure, the person or the person's health care provider will not know to take steps to attempt to lower the person's blood pressure. Similarly, a person having an infectious disease must be aware that he or she has the disease before that person can receive treatment for the disease.

Despite the benefits associated with timely and accurate determination of a person's physiological status, in many instances, people do not seek to obtain a diagnosis of their status as to one or more health-related conditions (e.g. infectious disease, pregnancy, or genetic mutation carrier). Such a failure to seek a diagnosis may be based on a fear that their confidentiality as to the health-related condition will not be fully preserved by the testing facility or a related party. For example, many people have a concern that information held by a laboratory testing facility may be stolen or accidentally released by the testing facility, thereby potentially making their health-related information available to individuals outside of the laboratory, or even to become generally available to the public at large.

Accordingly, improvements are desired in systems and methods for sample testing and for providing laboratory test results to a subject. In particular, improvements are desired in systems and methods for preserving the confidentiality of subjects before, during, and after sample testing.

For background art, the reader is directed to, for example, US 2005/0075543 A1, disclosing a method for providing test results to a subject and a computer readable medium having machine-executable code for implementing such a method.

### SUMMARY

Provided herein are systems and methods for ordering laboratory tests and providing the results of laboratory tests to a subject. With systems and methods provided herein, a subject may order a laboratory test, have the test performed, and receive results of the laboratory test without needing to provide information relating to his or her identity.

According to the invention, provided herein is a method for providing laboratory test results to a subject as defined in claim 1.

According to the invention, provided herein is a system for providing laboratory test results to a subject as defined in claim 11.

In embodiments, provided herein is a non-transitory computer-readable medium comprising machine-executable code for implementing a method for providing laboratory test results to a subject as specified above.

In embodiments, a LACS provided herein may comprise a server. The server may be, for example, a dedicated server or a component of a computer containing a user interface.

In embodiments, a LACS provided herein may be accessible via a network.

In embodiments, in systems and methods provided herein, a subject may enter a request for a laboratory test on a personal computing device operably connected to a LACS via a network.

In embodiments, a network provided herein may be the Internet.

In embodiments, a personal computing device provided herein may be a personal computer or a smart phone.

In embodiments, a laboratory test provided herein may be for an infectious disease, a genetic disease or condition, or pregnancy status. An infectious disease may be a sexually-transmitted disease.

In embodiments, in systems and methods provided herein involving a request from a subject for a laboratory test, the request from the subject is not associated with information relating to the identity of the subject.

In embodiments, information relating to the identity of a subject may comprise the subject's first name, middle name, last name, social security number, date of birth, age, home address, work address, driver's licence number, passport number, medical record number, and credit card number.

In embodiments, in systems and methods provided herein involving a request from a subject for a laboratory test, the request from the subject is associated with no more than 3, 2, 1, or 0 of any one or more of the subject's first name, middle name, last name, social security number, date of birth, age, home address, work address, driver's licence number, passport number, medical record number, and credit card number. For example, a request from a subject for a laboratory test may be associated with no more than 2 of the subject's first name, last name, date of birth, and social security number. In another example, a request from a subject for a laboratory test may be associated with no more than 1 of the subject's credit card number, home address, and last name.

In embodiments, an identifier code provided herein may comprise alphabetic characters, numeric characters, a bar code, an image, or a combination thereof.

A sample vessel provided herein is directly or indirectly associated with an identifier code or a related code.

In embodiments, in systems and methods provided herein, a laboratory technician may be involved in obtaining a sample from a subject.

According to the invention, in systems and methods provided herein, a sample vessel is handled by a laboratory technician during at least one of: i) a step of obtaining at a sample processing device a vessel containing a biological sample from the subject or ii) a step of processing a biological sample on a sample processing device according to a laboratory test requested by the subject. In embodiments in which a laboratory technician handles a sample vessel during one of the above steps, the technician does not know the type of laboratory test to be performed with the sample from the subject.

In systems and methods provided herein, prior to a biological sample in a sample vessel being processed on a sample processing device, the sample vessel containing the biological sample may be inserted into a cartridge containing reagents for performing two or more different laboratory tests.

In embodiments, in systems and methods provided herein involving a cartridge containing a biological sample and reagents for two or more different laboratory tests, not all of the laboratory tests which are supported by the reagents of the cartridge are performed on the sample in the cartridge.

In embodiments, in systems and methods provided herein, a cartridge may contain reagents for performing at least n different laboratory tests, and no more than n-1 laboratory tests are performed with the biological sample. In embodiments, n may be 500, 300, 100, 50, 25, 20, 15, 10, 9, 8, 7, 6, 5, 4, 3, or 2.

In embodiments, in systems and methods provided herein, information transmitted to a LACS may comprise raw data from a sample processing device or final laboratory test results.

In embodiments, in systems and methods provided herein involving a personal computing device, LACS and sample processing device, at least two or all three of the personal computing device, LACS, and sample processing device are located in the same room or building.

In embodiments, in systems and methods provided herein involving a personal computing device, LACS and sample processing device, at least two or all three of the personal computing device, LACS, and sample processing device are physically attached to each other or share a housing.

In embodiments, in systems and methods provided herein involving a personal computing device, LACS and sample processing device, at least two or all three of the personal computing device, LACS, and sample processing device are located in different locations (e.g. rooms, buildings, campuses, etc.). In embodiments, in systems and methods provided herein involving a personal computing device, LACS and sample processing device, at least two or all three of the personal computing device, LACS, and sample processing device are separated by at least 1, 10, 100, 1000, or 10,000 metres from each other.

In embodiments, in systems and methods provided herein, a LACS may receive a payment by a subject for a laboratory test. In embodiments, the payment may be made with a pre-paid card.

In embodiments, in systems and methods provided herein, a LACS does not store any information relating to the identity of the subject during the course of a system or method for providing laboratory test results to a subject.

In embodiments, in systems and methods provided herein, the systems may comprise a personal computing device, wherein the personal computing device is operably connected to a LACS via a network.

In embodiments, in systems and methods provided herein, a sample processing device may be configured to receive information regarding an identifier code or a related code on or associated with a sample vessel containing a biological sample.

In embodiments, in systems and methods provided herein, a sample processing device may comprise or be operably connected to a keypad for receiving alphabetic, numeric, or alphanumeric codes. In embodiments, in systems and methods provided herein, a sample processing device may comprise or be operably connected to an optical scanner, RFID reader, or magnetic strip reader for reading identifier code or related codes.

In embodiments, in systems and methods provided herein, a sample processing device may be configured to receive a cartridge comprising a biological sample from a subject and reagents for performing one or more laboratory tests.

In embodiments, in systems and methods provided herein, a sample processing device may comprise a pipette and may be configured to dilute a biological sample.

A system provided herein comprises a vessel containing a biological sample from a subject. The vessel is directly or indirectly associated with an identifier code or a related code.

A system provided herein comprises a cartridge containing reagents for performing two or more different laboratory tests and which may comprise a biological sample from the subj ect.

In embodiments, in a non-transitory computer-readable medium provided herein, the medium is in a server or a hard drive.

In embodiments, provided herein is a non-transitory computer readable medium comprising machine-executable code for implementing any method provided herein. The non-transitory computer readable medium comprising machine-executable code may be part of any system provided herein.

In embodiments, one or more steps of methods provided herein may be performed over a network. Various computing devices provided herein such as a personal computing device, a laboratory associated computer system, and a sample processing device may be operatively connected through wired or wireless connections to a network. The network may permit data communications between two or more devices or parties. For example, the network may permit a subject to use a personal computing device to request a laboratory test from a laboratory associated computer system (LACS). In another example, the network may permit a subject to receive from a LACS an identifier code for a laboratory test. In another example, the network may permit the subject to use a personal computing device to provide an identifier code or a related code to a LACS, and to receive the results of the laboratory test from the LACS. In another example, the network may permit a LACS to provide a protocol for processing a sample to a sample processing device. In another example, the network may permit a sample processing device to transmit information obtained from processing a sample from a subject to a LACS.

In embodiments, non-transitory tangible computer readable media comprising machine-executable code for implementing methods provided herein may be provided as a stand-alone and transportable product (e.g. a DVD, flash drive, magnetic tape, or other form of removable / insertable computer-readable media), such that the program or software stored thereon can be loaded onto one or more different computers, servers, or other computing devices, in order to implement one or more methods provided herein (or portions thereof). In other embodiments, non-transitory tangible computer readable media comprising machine-executable code for implementing methods provided herein may be provided as part of a computing device containing at least a data storage unit and a processor, such as a server or personal computer. In embodiments, a user may interact with software on a server via a client application running on a user device, which is coupled to the server via a network. For example, the software may include a WWW-based interface to allow a remote user / client to access laboratory test-related information. In embodiments, software running on a server may provide certain features to a user (e.g. a WWW-based interface), while performing various processes / operations on the server (e.g. correlation of laboratory test results with an identifier number).

In embodiments, provided herein is a laboratory test result apparatus taking the form of a machine readable storage medium (e.g., hard disk, CD, or other medium) (or multiple media) which contains a set of software instructions for execution by a processor for performing methods provided herein.

In embodiments, methods provided herein may be implemented using hardware, software, or a combination thereof. In embodiments, software code may be implemented using one or more processors, which may be distributed between one or more computing devices.

In embodiments, a method is provided for providing laboratory test result to a subject, wherein the subject's real life identity remains anonymous, even to the laboratory and/or its staff providing the test result.

In embodiments, a method is provided for providing laboratory test results to a subject, wherein the subject's real life identity remains anonymous to any third parties.

Other goals and advantages of the invention will be further appreciated and understood when considered in conjunction with the following description and accompanying drawings. While the following description may contain specific details describing particular embodiments of the invention, these should not be construed as limitations to the scope of the invention but rather as exemplifications of possible embodiments. For each aspect of the invention, many changes and modifications can be made within the scope of the invention.

### BRIEF DESCRIPTION OF THE DRAWINGS

In the drawings,
FIG. 1 shows a schematic depicting exemplary elements of systems and methods provided herein.
FIG. 2 shows exemplary steps of a method provided herein.

It is noted that the drawings and elements therein are not necessarily drawn to shape or scale. For example, the shape or scale of elements of the drawings may be simplified or modified for ease or clarity of presentation. It should further be understood that the drawings and elements therein are for exemplary illustrative purposes only, and are not to be construed as limiting in any way.

### DETAILED DESCRIPTION

Provided herein are systems and methods for ordering laboratory tests, performing laboratory tests, and for providing results of laboratory testing to a subject. The systems and methods permit a subject to anonymously have one or more laboratory tests performed, and to have the results transmitted to the subject. The systems and methods also permit a subject to select various amounts of information relating to the identity of a subject to be used during the process of ordering or performing a laboratory test, ranging from no information at all relating to his or her identity, to some information relating to his or her identity (e.g. a credit card number or a home address).

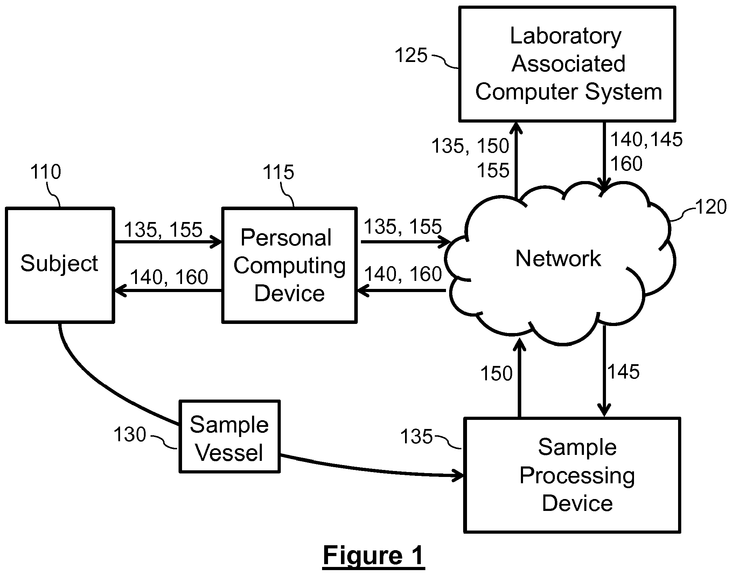

Embodiments of systems and methods provided herein may be described with reference to Figure 1. A subject 110 may wish to have a laboratory test performed, and to have little or no information related to his or her identity used during the laboratory testing procedure or related steps. In order to request a laboratory test, the subject may engage with a personal computing device 115. The subject may use the personal computing device 115 to transmit information to and to receive information from a laboratory associated computer system (LACS) 125. The personal computing device 115 and LACS 125 may be operably connected via a network 120. The LACS 125 may contain a processor and may run software which permits a subject 110 to place an order for a laboratory test 135. The software may permit the subject 110 to order a laboratory test 135 without providing identifying information about him or herself. In response to the subject's request for a laboratory test, the LACS 125 provides the subject 110 with an identifier code 140. An identifier code 140 may thus, in embodiments, for at least some portions of a laboratory testing process, take the place of personal identifying information about a subject, or may, in embodiments, be used in conjunction with personal identifying information about a subject. The subject 110 provides a biological sample, which is loaded into a sample vessel 130. The sample vessel 130 may be directly or indirectly associated with the identifier code or a related code. The sample vessel 130 containing the subject's biological sample may be provided at the location of a sample processing device 135, and sample from the sample vessel 130 may be introduced into the sample processing device 135. The sample processing device 135 may perform one or more steps of the laboratory test requested by the subject, using the sample from the subject. In embodiments, the LACS 125 may provide protocol information 145 to the sample processing device 135 for processing the sample from the subject, based on the particular laboratory test requested by the subject. Information from the one or more steps of the laboratory test with the sample is transmitted 150 from the sample processing device 135 to the LACS 125 via the network 120. The information may be the finalized laboratory test results or data which will undergo further analysis at the LACS 125 in order to determine the finalized test results. Ultimately, the LACS 125 may obtain the results of the test requested by the subject 110 (i.e. regardless of whether the test results are generated on the LACS 125 or transmitted to the LACS 125 from the sample processing device 135 or other device). Once the test results are available on the LACS 125, the subject 110 may request the results of the test 155. As illustrated in Fig. 1, the step of requesting test results is labeled "155" and the step of providing test results is labeled "160". The subject 110 may engage with the personal computing device 115 and use the personal computing device 115 to request the test results 155 from the LACS 125. To request the test results 155, the subject 110 provides the identifier code or a related code to the LACS 125. By providing the identifier code or a related code, the subject 110 does not need to enter identifying information about him or herself. In response to receiving the request for the test results 155, the LACS may provide the test results 160 to the subject 110 on the personal computing device 115. Accordingly, in embodiments of systems and methods provided herein, a subject may request a laboratory test from a laboratory, have the test performed, and receive the results of the test without providing identifying information about him or herself. In addition, in embodiments, a subject 110 may provide some personal identifying information about him or herself during a laboratory testing process. As used herein, a "subject" refers to any person that may be interested in obtaining a laboratory test result. Subjects include healthy individuals as well as individuals with one or more health problems.

As used herein, a "biological sample" refers to any naturally occurring material that may be part of or be produced by a subject, such as blood, serum, saliva, urine, gastric and digestive fluid, tears, stool, semen, vaginal fluid, interstitial fluids derived from tumorous tissue, ocular fluids, sweat, mucus, earwax, oil, glandular secretions, breath, spinal fluid, hair, fingernails, skin cells, cheek swab, gums swab, mouth swab, plasma, nasal swab or nasopharyngeal wash, spinal fluid, cerebrospinal fluid, tissue, throat swab, biopsy, placental fluid, amniotic fluid, cord blood, emphatic fluids, cavity fluids, sputum, pus, microbiota, meconium, breast milk or a combination of any one or more thereof. A "biological sample" may be a material that a subject is capable of readily obtaining from him or herself without the assistance of others (e.g. saliva, cheek swab, urine) or it may be a material that typically or always requires the assistance of one or more others to obtain (e.g. cerebrospinal fluid). With systems and methods provided herein, a subject may provide one or more biological samples. For example, in some circumstances, a subject may provide only a sample of saliva. In another example, a subject may provide a sample of saliva and a sample of blood. In another example, a subject may provide a sample of blood, where the blood sample from the subject is collected in two separate vessels. As used here, "a sample" refers to any number of samples and sample types from a subject, unless the context clearly provides otherwise.

As used herein, a "computing device" refers to any device or system which may collect, display, store, or process digital data. Computing devices may include, for example, servers, smartphones, personal computers, hard drives, and systems containing processors and data storage units.

A subject may wish to have a laboratory test performed, and to have little or no information related to his or her identity used during the laboratory testing procedure or related steps. Information relating to the identity of a subject can include, for example, any one or more of a subject's first name, middle name, last name, social security number, date of birth, age, home address, work address, driver's license number, passport number, medical record number, credit card number, or biometric information (e.g. information relating to a subject's finger or hand print, DNA, blood type or other blood characteristics, facial features, ear structure, iris structure, or retina structure). In embodiments, with systems and methods provided herein, a subject may provide no more than 3, 2, 1, or none of his or her: first name, middle name, last name, social security number, date of birth, age, home address, work address, driver's license number, passport number, medical record number, credit card number, or biometric information during the processing of ordering a laboratory test, having the test performed, or receiving the test results.

A subject may have access to a personal computing device. As used herein, a "personal computing device" refers to any computing device with which a user may interact to input or receive information. Typically, a personal computing device contains or is operably connected to one or more processors and one or more data storage units (e.g. main memory units or secondary storage units). Personal computing devices include desktop computers, laptop computers, tablet computers, and smart phones (e.g. Apple iPhone or Android-enabled phone). A personal computing device may be portable or non-portable / at a fixed location. A personal computing device may contain one or more user interfaces. User interfaces may provide information to a user, obtain inputs from a user, or both. A user interface may include a display, such as a cathode ray tube, plasma, liquid crystal display (LCD), or light-emitting diode (LED) - based display. In embodiments, a user interface may include a graphical user interface (GUI) configured to display information to a user on a display. A GUI may also be configured to receive information from a user, such as by capacitive or resistive touch-screen functions or through a computer mouse. In some situations, user interfaces may include a camera for obtaining video or still images, a microphone for capturing audible information (e.g., a subject's voice), speakers for providing audible information, a printer for printing information, or a projector for displaying images and/or video onto a viewing surface. Other user interfaces of a personal computing device may include, for example, a keyboard, touch pad, or a computer mouse. In embodiments, personal computing devices may include devices which are publically available, such as a computer terminal at a retail or health care provider location. In embodiments, a subject may use a publically-available personal computing device stationed at a drug store or other location to order a laboratory test.

Typically, a personal computing device is operably connected to a network. A network may be any structure which can support the operable connection of and data transfer between two or more network-enabled devices. The network may be a local area network (LAN) or a wide area network (WAN), and may include, for example, an intranet, an enterprise private network, the Internet, cellular, or satellite networks. The network may include, for example, one or more of wireless connections, wired connections, and fiber optic connections. As used herein, "network-enabled device" refers to an electronic device configured to connect to and communicate with one or more electronic devices with the aid of a network. Network-enabled devices (also referred to as "network devices" herein) may include, for example, computing devices, servers, smartphones, personal computers, mainframe computers, hard drives, and systems of computers. Network devices may connect to the network by wired or wireless technologies. For example, a network device may connect to the network via wired technologies such as a dial-up connection with a modem, a direct link such as TI, ISDN, cable, Firewire, USB, or Ethernet wire. In other examples, a network device may connect to the network via wireless technologies such as Bluetooth, RTM, infrared (IR), radio frequency (RF), ZigBee, Z-wave, wireless USB, code division multiple access (CDMA) or global system for mobile communications (GSM). In embodiments, data may be encrypted before it is transmitted over the network.

A subject may use a personal computing device to access a laboratory associated computer system (LACS). The personal computing device and LACS may be operably connected via a network. In other embodiments, the personal computing device and LACS may be directly linked, without an intervening network. The LACS may run software which contains instructions for performing one or more operations, such as permitting a subject to place an order for a laboratory test, providing a subject with an identifier code in response to a request for a laboratory test, providing protocol information to a sample processing device for processing a sample from a subject, receiving information from the testing of a sample from a sample processing device, and correlating the information with an identifier code for the sample, analyzing data from a sample processing device in order to determine test results resulting from one or more processing steps performed on the sample processing device with a sample from a subject, and providing a subject with test results in response to receiving a request for the test results from the subject.

The LACS may contain one or more processors and one or more data storage units.. The processor may be a hardware structure which performs computational operations of a computer program. In embodiments, a processor may carry out instructions stored in a tangible computer readable medium. The processor may contain one or more microprocessors. Data storage units may include, for example, main memory units and secondary storage units. A main memory unit is a structure for storage of digital information which typically uses volatile storage and is rapidly or directly accessible by a processor (e.g. random access memory (RAM)). A secondary storage unit is a structure for storage of digital information which typically uses non-volatile storage, and which typically has a much larger storage capacity than a main memory unit, but is less quickly accessible by a processor than is a main memory unit. An example of a secondary storage unit is a hard drive. In embodiments, a main memory unit or secondary storage unit may store non-transitory computer readable media, which may include, for example, code, logic, or instructions for performing methods provided herein. A LACS may have any number of processors or data storage units (e.g. 1, 2, 3, 4, 5, 6, 7, 8, 9, 10, 15, 20, 25, 50, 100, 1000, or more of any or each of the processors or data storage units). A LACS may also contain other components, such as a removable media drive (which may accept, for example, CDs, DVDs, floppy disks, or magnetic tape-based storage), input-output (I/O) channels, buses, network interfaces (wired or wireless structures for facilitating data transfer between a server and a network), or power supplies. A LACS may have a variety of different shapes and structures. In an embodiment, a LACS may be or contain a server. A LACS which is a server may be a dedicated server, or it may be part of a computer which contains other features (e.g. a monitor, peripherals, etc.). In embodiments, a LACS may contain two or more servers. A LACS may contain one or more primary servers and one or more caching servers, where the caching servers store content that is frequently accessed by users. In some embodiments, a LACS may be part of, for example, a personal computer or a smart phone.

A system provided herein may contain non-transitory tangible computer readable media (e.g. in a LACS, sample processing device, or personal computing device). Computer readable media can be any available media which can be directly or indirectly accessed by a LACS or other computing device of a system provided herein. Computer readable media may include volatile and nonvolatile media, as well as removable and non-removable media. Computer readable media may be implemented in any method or technology for storage of information, such as computer readable instructions, data structures, program modules, or other data. Computer storage media may include, but is not limited to, RAM, ROM, EEPROM, flash memory or other memory technology, CD-ROM, digital versatile disks (DVDs) or other optical disk storage, magnetic cassettes, magnetic tape, magnetic disk storage or other magnetic storage device, or any other medium which can be used to store information and which may be accessed by a processor or computing device.

In embodiments, a main memory unit or secondary storage unit may store transitory computer readable media, which may include, for example, code, logic, or instructions for performing methods provided herein provided via metal, fiber-optic, or other fixed communication lines, or via wireless or other communication means. In embodiments, a main memory unit or secondary storage unit may store protocols or other instructions for performing methods provided herein; such protocols or other instructions may be fixed (e.g., may be unmodifiable) or may be capable of modification. In embodiments, modification of protocols or other instructions may include modifications to update or improve the protocol or instructions; modifications to correct the protocol or instructions; modifications to adjust the protocol or instructions in view of environmental conditions; modifications to adjust the protocol or instructions for use with a particular subject (e.g., in view of subject age, sex, medical condition, or other characteristic); modifications to adjust the protocol or instructions for use with a particular sample processing device (e.g., in view of construction or performance characteristics of the device); or other modifications.

Using a personal computing device, a subject may access a LACS. The LACS may provide an interface on the personal computing device that allows a subject to select one or more laboratory tests to be performed with a biological sample from a subject. The laboratory tests which may be selected may relate to a status or condition of a subject, and may test for, for example, an infectious disease, a genetic disease or condition, a sexually-transmitted disease, a respiratory disease, a degenerative disease, a dementing disease or condition, a malignant disease or condition, an autoimmune disease or condition, a chronic disease or condition, an acute disease or condition, an injury, a childhood disease, an adult-onset disease, an illegal drug or drug of abuse, pregnancy status, a genetic profile, a proteomic profile, or a condition or test which is a combination of any two or more thereof. In embodiments, two or more different laboratory tests may be performed with a single sample from a subject. A subject 110 may select 1, 2, 3, 4, 5, 6, 7, 8, 9, 10, 15, 20, 25, 50, 100, or more laboratory tests be performed with a sample from a subject. Two or more different laboratory tests may also be performed with two or more samples from a subject (e.g. blood and urine). As used herein, unless the context clearly dictates otherwise, a reference to "a test" or "the test" and the like herein includes both a single test and also two or more tests. In some embodiments, a subject 110 may individually select one or more specific different laboratory tests to be performed with a sample from the subject. In other embodiments, a subject 110 may select a test panel which includes two or more laboratory tests, where the specific tests in the test-panel are chosen by the laboratory or other entity. For example, a subject may select a "sexually transmitted disease panel", where the laboratory or other entity decides the specific tests which are to be performed as part of the panel. An exemplary sexually transmitted disease panel may include, for example, tests for HIV, gonorrhea, chlamydia, and syphilis.

In embodiments, infectious diseases which may be tested for may include, for example, any one or more of: HIV / AIDS, chancroid, chickenpox, chlamydia, cholera, cytomegalovirus, diphtheria, enterobiasis (pinworm), typhus, giardiasis, gonorrhea, hepatitis A, hepatitis B, hepatitis C, hepatitis D, hepatitis E, herpes simplex, human papillomavirus (HPV), Epstein-Barr virus (mononucleosis), leprosy, Lyme disease, malaria, measles, mumps, pelvic inflammatory disease, pertussis, pneumonia, polio, rabies, rotavirus infection, rubella, SARS, shingles, sleeping sickness, syphilis, tuberculosis, tularemia, West Nile Fever, Ebola fever, Marburg fever, and yellow fever. In testing for any of the above, the test may involve, for example, assaying for a nucleic acid (e.g. DNA or RNA) or protein from the respective infectious pathogen.

In embodiments, infectious diseases which may be tested for are respiratory diseases. In embodiments, tests for respiratory diseases may include tests for one or more of the group of respiratory diseases consisting of adenovirus B, adenovirus C, adenovirus E, Bordetella pertussis, mycobacterium tuberculosis (MTB), Staphylococcus aureus, Methicillin-Resistant Staphylococcus aureus (MRSA), Group A streptococcus, and Group B streptococcus. In further embodiments, tests for respiratory diseases may include tests for one or more of the group of respiratory diseases consisting of adenovirus B, adenovirus C, adenovirus E, Bordetella pertussis, Bordetella parapertussis, mycobacterium tuberculosis (MTB), Staphylococcus aureus, Methicillin-Resistant Staphylococcus aureus (MRSA), Group A streptococcus, Group B streptococcus, Moraxella catarrhais, Enterobacter aerogenes, Haemophilus parainfluenzae, Metapneumo Virus, Streptococcus pneumonia, Parainfluenza Virus 1, Parainfluenza Virus 2, Parainfluenza Virus 3, Coronavirus OC43, Coronavirus NL63, Coronavirus MERS, Coronavirus HKU1, Coronavirus 229E, Klibsiella pneumonia phoE, Klebsiella pneumonia KPC, Bocavirus type 2,4, and Bocavirus type 1,3.

In embodiments, infectious diseases may be influenza (either influenza A or influenza B). Tests for influenza include one or more of the group consisting of the following forms of influenza: H1N1 (seasonal), H1N1 (novel), H3N2, H7N9 (hemagglutinin gene marker (HA) and neuraminidase gene (NA)), and H5N1. In embodiments, tests for influenza may include tests for influenza markers, such as, e.g., influenza Matrix Protein markers, or influenza neuraminidase protein markers, or may be influenza hemagglutinin markers, or other influenza markers.

In embodiments, infectious diseases which may be tested for are sexually transmitted diseases. Sexually transmitted disease may include, for example, any one or more of: HIV/AIDS (e.g. HIV-1 (e.g. group M), HIV-2 (e.g. group A, B)), syphilis, gonorrhea, human papillomavirus (HPV), chancroid, chlamydia, cytomegalovirus, hepatitis (e.g. B, C, D) pelvic inflammatory disease, pubic lice, scabies, and infections caused by some herpes viruses (herpes simplex virus (HSV)). In testing for any of the above, the test may involve, for example, assaying for a nucleic acid (e.g. DNA or RNA) or protein from the respective infectious pathogen.

In embodiments, a sample from a subject may be tested in order to determine whether the subject has a particular genetic mutation, to identify the particular variant of a polymorphic gene a subject possesses, or to identify two or more features of a subject's genome. As used herein "genetic disease or condition" refers to a disease caused by, or whose incidence is highly correlated with, a person's genetic makeup, whether inherited or due to mutation. Genetic diseases and genetic conditions may include, for example, many cancers (e.g., breast and ovarian cancer linked, e.g., to the presence of mutations in the BRCA1 or BRCA2 gene); some forms of Alzheimer's disease (e.g., those linked to mutations in the amyloid precursor gene protein, or in a presenilin gene, or other genes); cystic fibrosis; Down Syndrome; Duchenne and Becker Muscular Dystrophy; Niemann-Pick disease; Tay-Sachs disease; Bloom's Syndrome; Huntington's disease; celiac disease (which is also an autoimmune disease); Noonan Syndrome; Amyotrophic Lateral Sclerosis; Marfan Syndrome; Sickle-Cell Anemia; Thalassemia; Retinitis Pigmentosa; and other genetic and inherited diseases and conditions.

In embodiments, degenerative diseases which may be tested for may include, for example, any one or more of: amyotrophic lateral sclerosis (ALS), Alzheimer's disease, Parkinson's disease, Friedreich's ataxia, multiple sclerosis, muscular dystrophy, diabetes, inflammatory bowel disease, rheumatoid arthritis, Huntington's disease, chronic obstructive pulmonary disease (COPD), Ehlers-Danlos syndrome, and Marfan's syndrome.

In embodiments, dementing diseases which may be tested for may include, for example, any one or more of: Alzheimer's disease, vascular dementia, frontotemporal dementia, semantic dementia, dementia with Lewy bodies, Huntington's disease, chronic traumatic encephalopathy, SCA17, adrenoleukodystrophy, Gaucher's disease, metachromatic leukodystrophy, Wilson's disease, and Creutzfeldt-Jakob disease.

In embodiments, malignant diseases which may be tested for may include, for example, any one or more types of cancer such as: bladder cancer, breast cancer, colon cancer, rectal cancer, endometrial cancer, renal cell cancer, leukemia, lung cancer, melanoma, lymphoma, pancreatic cancer, prostate cancer, thyroid cancer, stomach cancer, testicular cancer, ovarian cancer, and skin cancer.

As used herein, the term "autoimmune disease" refers to a disease whose symptoms are due to loss of function or inflammation caused by a subject's own immune system. Autoimmune diseases which may be tested for may include, for example, any one or more of: rheumatoid arthritis, multiple sclerosis, myasthenia gravis, type I diabetes, pernicious anemia, Sjogren's syndrome, lupus erythematosis, Grave's disease, celiac disease, and Addison's disease.

In embodiments, illegal drugs or drugs of abuse which may be tested for may include, for example, any one or more of alcohol, cathinones, GHB, rohypnol, ketamine, MDMA (ecstasy), methamphetamine, LSD (acid), cocaine, heroin, marijuana, phencyclidine, salvia, anabolic steroids, and nicotine.

Laboratory tests may be performed by any appropriate method for assessing the analyte or condition of interest, such as protein-based testing, nucleic acid-based testing, immunoassays, receptor-based assays, cytometric assays, colorimetric assays, enzymatic assays, electrophoretic assays, electrochemical assays, spectroscopic assays, chromatographic assays, microscopic assays, topographic assays, calorimetric assays, turbidmetric assays, agglutination assays, radioisotope assays, viscometric assays, coagulation assays, clotting time assays, protein synthesis assays, histological assays, culture assays, osmolarity assays, and combinations thereof.

In embodiments, a subject may be able to interact with LACS software through a client application from the LACS running on a personal computing device. A client application may be or include, for example, a World Wide Web (WWW)-based interface. A WWW-based interface may be provided, for example, at a specific URL (e.g. a web page), which a subject may access via the network through a personal computing device. A subject may request a WWW-based interface at a specific URL, and the content may be delivered to the subject's personal computing device from the LACS. In embodiments, a subject may input information on a WWW-based interface, and the information may be transmitted to the LACS. In embodiments, a WWW-based interface may permit a user to log in to one or more web pages associated with an identifier code, to permit the user to access one or more interconnected web pages containing information associated with the identifier code. In embodiments, a WWW-based interface may provide laboratory test-related information. With the WWW-based interface, a user may optionally be able to, for example, request a laboratory test be performed, request the results of a laboratory test, or pay for a laboratory test.

In order for a subject to order a laboratory test from the LACS, a subject is not required to provide identifying information about him or herself. For example, a subject may request one or more laboratory test from the LACS without providing his or her first name, middle name, last name, social security number, date of birth, age, home address, work address, driver's license number, passport number, insurance ID number, medical record number, or biometric information during the ordering process. Alternatively, in embodiments, a subject may provide no more than 3, 2, or, 1 of the above during the ordering process. In order to request a laboratory test from the LACS without providing identifying information, the LACS interface on the personal computing device may permit a subject to, for example, request a laboratory test without first creating a user account containing personal information or otherwise providing personal information. The LACS may provide an interface on a personal computing device where a subject can select one or more laboratory tests to be performed with a sample from the subject (e.g. a drop-down or checkbox-based menu).

In embodiments, before, during, or after requesting one or more laboratory tests from the LACS, the subject may receive an identifier code from the LACS. For example, a subject may request one or more laboratory tests from the LACS, and in response, the LACS may generate an identifier code which is associated with the selected laboratory test, and then the identifier code may be provided to the subject. In another example, at the start of the laboratory test ordering processes, a subject may be provided with an identifier code from the LACS. Then, once the subject finishes ordering the laboratory test(s), the selected laboratory test(s) are associated by the LACS with the identifier code previously provided to the subject. In embodiments, an identifier code may be newly generated at the time of a request for a laboratory test from a subject. In other embodiments, the LACS may store identifier codes which are ready for use, and provide a previously stored identifier code to a subject upon a subject's request for a laboratory test. Regardless of the timing of the generation of an identifier code or the timing of its distribution to a subject, in systems and methods provided herein, an identifier code is associated with one or more laboratory tests to be performed on a biological sample from a subject, and the identifier code is provided to a subject. A data storage unit in or associated with the LACS may store issued identifier codes, and the requested laboratory test(s) that each identifier code is associated with.

An identifier code may comprise, for example, a symbol, such as a bar code, Quick Response code (QR code), or arbitrary symbol; a number (i.e., a numeric code); a series of letters (i.e., an alphabetic code); a combination of numbers and letters (i.e., an alphanumeric code); a combination of symbols, numbers, and letters; or other code. An identifier code may be, for example, displayed on a cell-phone, displayed on a tablet, displayed a computer screen, printed on paper, or presented by other methods. In embodiments, a subject may receive a hard-copy of the identifier code or receive a tangible object containing the identifier code (e.g. a subject may receive a sticker or paper printed with an identifier code on it or may receive a card with a magnetic stripe containing the identifier code). An identifier code may be machine readable, may be readable by a human, or readable by both machine and human. Where visible to a human or to a scanner, camera, or other light-sensing device, an identifier code may be black and white, may comprise gray, may comprise colors, and may comprise a combination thereof. An identifier code may comprise visual elements; may comprise audio elements; may comprise electrical elements; may comprise magnetic elements; may comprise electromagnetic signals or elements; may comprise mechanical elements; and may comprise combinations thereof. For example, an identifier code that comprises audio elements may comprise the generation of, or the reception of, a sound (e.g., a pitch, or series of pitches, or combination of pitches). For example, an identifier code that comprises magnetic elements may comprise the generation of, or the reception of, a magnetic field, or the placement of a magnet on or near a sensor. For example, an identifier code that comprises electromagnetic elements may comprise the generation of, or the reception of, an electromagnetic signal (e.g., a signal comprising radio, microwave, infrared, visual, ultraviolet, or other electromagnetic energy). In embodiments, an identifier code may comprise mechanical elements, including elements that require physically mating or fitting one object to another (such as, e.g., a key which fits in a lock, a card which slides in a slot, a tag which is configured to lodge in a tag reader, or other mechanical elements). An identifier code may comprise a combination of mechanical and other elements (e.g., a card comprising a magnetic strip, in which the card must slide in a slot in order that a magnetic code may be read by a card-reader). An identifier code may comprise, or the provision of an identifier code may require, a timing signal, a Global Positioning System (GPS) signal, a video signal, or other signal, and may comprise a combination thereof. In embodiments in which a subject is to receive a hard-copy of the identifier code or tangible object containing the identifier code, in some circumstances, a subject may order a test on a computing device at a location where the subject can also receive the tangible object containing the identifier code. For example, in embodiments, a subject may order a laboratory test via a personal computing device located in a retail location (e.g. a drug store), and upon ordering the test, the subject may receive the tangible object containing the identifier code from a person or device at the retail location (e.g. a device may print out a sticker containing the identifier code or an employee may hand the subject a card containing a magnetic strip containing the identifier code).

In embodiments, an identifier code may comprise a plurality of codes, or a plurality of code elements. An identifier code comprising a plurality of codes, or a plurality of code elements, may include any number of codes, or any number of code elements. For example, an identifier code comprising a plurality of codes may comprise two codes; or three codes; or four codes; or more codes. For example, an identifier code comprising a plurality of code elements may comprise any number of code elements; for example, a numeric code may comprise 1, 2, 3, 4, 5, 6, 7, 8, 9, 10, 11, 12, 13, 14, 15, 20, 30, 40, 50, or more digits, and an alphabetic code may comprise 1, 2, 3, 4, 5, 6, 7, 8, 9, 10, 11, 12, 13, 14, 15, 20, 30, 40, 50, or more letters. For example, an identifier code comprising a plurality of codes may comprise an alphanumeric code together with a bar code. In another example, an identifier code comprising a plurality of codes may comprise an alphanumeric code together with a QR code. In another example, an identifier code comprising a plurality of codes may comprise an alphanumeric code comprising characters (e.g., digits and letters) written in different colors together with an RFID code. In another example, an identifier code comprising a plurality of codes may comprise a numeric code, a mechanical code (e.g., a card), a magnetic code (e.g., a magnetic strip), and a QR code. An identifier code comprising a plurality of codes may comprise any combination of codes.

In embodiments, an identifier code may contain information which directly relates to the test(s) requested by the subject who received the identifier code. For example, in a system, different tests may be associated with different numeric codes (e.g. a test for chickenpox may be "5879" and a test for mumps may be "3442"). In such a system, an identifier code containing "5879-3442", would provide that the sample was to be tested for chickenpox and mumps. In other embodiments, the composition of the identifier code may not directly relate to the test(s) requested by the subject who received the identifier code. For example, each new request in the LACS system might be issued a numeric identifier code based on the order in which the request was received at the LACS, or other protocol. In such a circumstance, a data storage unit in the LACS may store which test(s) are associated with which identifier code, and content of the identifier code itself may not contain any information directly relating to the test(s) requested relating to the identifier code. In some embodiments, an identifier code may have characteristics of both of the above (i.e. it may contain some code elements which directly relate to a test to be performed, and some code elements which do not directly related to a test to be performed).

In embodiments, an identifier code may be capable of being displayed on a personal computing device (e.g. a smart phone, laptop computer, or tablet computer). In embodiments, an identifier code can be downloaded onto a computing device, so that a subject can access the identifier code independent of whether the device is connected to the network. In embodiments, the LACS may provide a link to a specific URL (e.g. a webpage) where a subject's identifier code is provided. An identifier code may be capable of being printed on paper, so that, for example, a subject may obtain a sheet of paper which contains the identifier code.

Accordingly, in embodiments a subject may receive an identifier code, order tests, have tests performed, and receive test results without providing identifying information about him or herself. For example, a subject may receive an identifier code, order tests, have tests performed, and receive test results without providing his or her first name, middle name, last name, social security number, date of birth, age, home address, work address, driver's license number, passport number, insurance ID number, medical record number, or biometric information during the ordering process. In embodiments, a subject may provide no more than 3, 2, or, 1 of the above and may receive an identifier code, order tests, have tests performed, and receive test results. In embodiments, where a subject provides some or all of such identifying information about him or herself, all of, some of, or none of the identifying information may be associated with the identifier code provided to the subject.

A subject who has requested one or more laboratory tests and who has received an identifier code for the tests provides a biological sample to be tested. Typically, a biological sample obtained from a subject will be directed into a vessel. As used herein, a "vessel" refers to any structure which can receive and at least temporarily store a biological sample. Thus, as used herein, a "vessel" may refer to, for example, a tube, a cup, a swab holder, or a structure having an accessible channel. In embodiments, when selecting a laboratory test with a LACS, a subject may also select a method to obtain a vessel or to provide a biological sample to a vessel.

In embodiments, a subject may readily be able to obtain a biological sample from him or herself without the assistance of others, and to direct the sample into a sample vessel. Such biological samples may include, for example, saliva, cheek cells, or urine. In other embodiments, a subject may typically or always require the assistance of at least one other person to obtain the sample and to direct it into a sample vessel. Such biological samples may include, for instance, cerebrospinal fluid. In embodiments, a biological sample may be blood. In some circumstances, a subject may be able to collect blood from him or herself without the assistance of others, and in some circumstances, blood may be collected from a subject with the assistance of another person.

A vessel which accepts a sample from a subject may be directly or indirectly associated with the subject's identifier code, or a related code. A vessel may be directly associated with a code, if, for example, the code is physically attached to the vessel (e.g. if the code is capable of being printed on a label, and a label containing the code is adhered to the vessel). A vessel may be indirectly associated with a code, if, for example, the code is physically associated with a container which may store or transport the vessel (e.g. if the code is capable of being printed on a label, a label containing the code is adhered to a box, and the vessel is stored inside of the box). In another example, a vessel may be indirectly associated with a code, if the code is physically associated with a container which may store or transport the vessel, and the position of the vessel inside of the box is known or stored as information associated with the code. Any other suitable method for linking an identifier code or a related code to a vessel may be used with systems and methods provided herein.

As explained above, a vessel which accepts a sample from a subject may be directly or indirectly associated with the subject's identifier code for the test(s) requested by the subject, or a related code. As used herein, a "related code" to an identifier code is any code which the LACS or other system can connect with the original identifier code (e.g. through operation of an algorithm in a processor in the LACS). A related code need not have any of the same characteristics as the corresponding identifier code, as long as it can be connected to the identifier code. For instance, the original identifier code may be a numeric code of 20 digits. Before, during, or after the generation of the original identifier code, the LACS may generate a related alphabetic code of 15 letters. The LACS may store or associate the related alphabetic code of 15 letters with the identifier code of 20 digits. Thus, if the LACS receives information or instructions relating to either the original identifier code (of 20 digits) or the related code (of 15 letters), it may group the information or instructions with the same sample and laboratory tests. Use of an identifier code plus one or more related codes may provide various advantages, such as to increase the security or organization of the system.

Use of an identifier code plus one or more related codes may increase the security of the system, for example, by limiting which codes may be used to access information relating to a given test. For example, a subject may request a laboratory test as described herein, and may be issued an identifier code for the requested test. Then, the vessel which receives the sample from the subject may be associated with a related code (rather than the actual identifier code issued to the subject). The system may be configured such that the identifier code may be used by the subject to look up the results of the laboratory test, but the related code cannot be used to obtain the results of the laboratory test. This may be useful, for example, so that a person (e.g. a laboratory technician) who handles or otherwise interacts with the vessel cannot use the related code to look up the test results from the sample in the vessel, but the sample vessel can still be associated with the test ordered by the subject.

Use of an identifier code plus one or more related codes may facilitate tracking of samples, for example, in circumstances in which two or more samples from a subject are to be collected and used for laboratory testing (e.g. a blood sample and a urine sample). In such circumstances, it may be desirable to permit the subject to access all of his or her laboratory results in the LACS by using the same identifier code (including the results obtained from the different samples in the different vessels). However, it may also be desirable to use different codes with each of the two or more vessels which contain sample from the subject, in order to keep track of the different samples and vessels. In this circumstance, for example, each of the vessels may be labeled with a different code which is related to the original identifier code. By using related codes, the different vessels can be clearly labeled and differentiated, but also still associated with the original identifier code.

In embodiments, if a subject provides information relating to his or her identity, a sample vessel may be directly or indirectly associated with some or all of the information (e.g. a subject's date of birth). Use of some information relating to the identity of a subject to label a vessel may, for example, help a subject have confidence that he or she has the correct vessel.

A subject obtains a vessel into which a biological sample may be loaded. The vessel is directly or indirectly labeled with the identifier code, or a related code. Typically, the subject may obtain a vessel for loading with a biological sample when the sample to be collected is of a type which a subject may readily obtain from him or herself without the assistance of others. A vessel may be provided to a subject in one or more different ways. In some embodiments, a subject may pick up a vessel at a designated pick-up location. Designated locations may include any suitable location for storage and pick up of vessels. In embodiments, a vessel may be picked up by a subject at a point of service location. Point of service locations may include locations where a subject may receive a service (e.g. testing, monitoring, treatment, diagnosis, guidance, sample collection, ID verification, medical services, non-medical services, etc.), and may include, without limitation, a subject's home, a subject's business, the location of a healthcare provider (e.g., doctor), hospitals, emergency rooms, operating rooms, clinics, health care professionals' offices, laboratories, retailers [e.g. pharmacies (e.g., retail pharmacy, clinical pharmacy, hospital pharmacy), drugstores, supermarkets, grocers, etc.], transportation vehicles (e.g. car, boat, truck, bus, airplane, motorcycle, ambulance, mobile unit, fire engine/truck, emergency vehicle, law enforcement vehicle, police car, or other vehicle configured to transport a subject from one point to another, etc.), traveling medical care units, mobile units, schools, day-care centers, security screening locations, combat locations, health assisted living residences, government offices, office buildings, tents, bodily fluid sample acquisition sites (e.g. blood collection centers), sites at or near an entrance to a location that a subject may wish to access, sites on or near a device that a subject may wish to access (e.g., the location of a computer if the subject wishes to access the computer), a location where a sample processing device receives a sample, or any other point of service location described elsewhere herein.

In embodiments, a vessel for pick-up may be directly or indirectly labeled with an identifier code or a related code at the time of pick-up of the vessel. In other embodiments, a vessel for pick-up may not be labeled with an identifier code or related code at the time of pick-up of the vessel, and instead, the subject may label the vessel with the identifier code or related code after he or she has obtained the vessel. In circumstances in which a vessel is not labeled with an identifier code or related code at the time of the pick-up of the vessel, in embodiments, the subject may be instructed as to what type of vessel to obtain at the pick-up location. In some embodiments, only a single type of vessel is available at a pick-up location. In other embodiments, two or more different types of vessel are available at a pick-up location (e.g. one type of vessel for accepting saliva samples, and another type of vessel for accepting urine samples), and a subject is instructed during the laboratory test ordering process as to what type of vessel to acquire at the pick-up location. In other embodiments, a subject may provide a mailing address to the LACS to where the sample vessel is to be mailed to the subject. The address may be any address where the subject is capable of picking up mail, such as a P.O. Box, home, or business address. Optionally, at the subject's request, the vessel can be mailed to the subject's home address. If the subject chooses to have the vessel mailed to his or her home address, a subject may still choose to not include his or her name in the address, or to include a false name as the recipient. Sample vessels which are mailed to a location may be pre-labeled with an identifier code or related code, or a subject may later label a vessel with an identifier code or related code after receiving the vessel. In another embodiment, subject may purchase at a retail location (e.g. drug store, supermarket, etc.) a kit which contains the appropriate vessel(s) for a laboratory test(s) associated with the kit. For example, kits for certain laboratory tests may contain a vessel for collecting urine, and kits for certain other laboratory tests may contain a vessel for collecting a cheek swab. Optionally, the kit may further contain a code or other form of information which a subject may enter on an interface with the LACS, to provide payment for an ordered laboratory test. In such a circumstance, for example, the purchase of the kit may include the price for the laboratory test. Thus, the purchasing of a kit containing a sample vessel may facilitate both the distribution of a sample vessel to a subject, as well as the collection of payment for a laboratory test. In some embodiments, a vessel in a kit may be pre-labeled with an identifier code or a related code. The subject may enter this identifier code or related code, for example, when ordering a laboratory test with the LACS. In other embodiments, a subject may label a vessel from a kit with an identifier code or a related code after the subject has placed an order with the LACS for a laboratory test, and received an identifier code from the LACS. When purchasing a kit containing a sample vessel, a subject may choose to use any form of payment. For example, a subject seeking the highest level of confidentiality may pay for a kit with cash or a money order, whereas a different subject may choose pay for a kit with a personal credit card or debit card. While the payment for a kit with a personal credit or debit card should still preserve the confidentiality of the subject, it may not provide the same level of confidentiality as a cash or other payment which cannot be easily traced to the identity of the payer. A subject may also obtain a sample vessel by any other suitable method.

When a subject obtains a vessel for sample self-collection, after the subject has collected the sample, the subject may direct the vessel to a location where a sample processing device is located. The subject may direct a vessel to a location of a sample processing device in various ways. For example, the subject may mail the vessel to the location of a sample processing device via any appropriate carrier (e.g. United States Postal Service, FedEx, UPS, etc.). In another example, the subject may drop off the vessel at a designated location for receiving vessels (e.g. a point of service location). In some situations, the subject may drop off the vessel at the same location from which the subject picked up the vessel. In embodiments, a subject may obtain a vessel from a location, load a self-collected sample into the vessel, and then return the vessel to the same location, all within a short period of time (e.g. within 1 hour, 30 min, 20 min, 10 min, 5 min, or less). For example, a subject may pick up a vessel for saliva collection at a point of service location, collect a saliva sample into the vessel, and then return the vessel to the same point of service location during the course of a single short visit to a point of service location.

In embodiments, a subject may provide a biological sample which is loaded into a vessel. In embodiments, for certain sample types, a subject may typically or always require the assistance of at least one other person in order to obtain the sample for loading into a vessel. As used herein, a person who assists in obtaining a sample from a subject may be referred to as a "sample collection technician". A sample collection technician is a type of "laboratory technician". A sample collection technician may be any person having sufficient training for obtaining a relevant type of biological sample, and may include, for example, phlebotomists, nurses, emergency medical technicians, and medical doctors. A sample collection technician may be located at (and a subject may provide sample for analysis at) a point of service location, such as a retail location or health clinic.

In situations in which a sample collection technician is involved in collecting the sample from the subject, a subject may not independently have possession of the vessel for a significant period of time before or after the sample is loaded into the vessel (e.g. the vessel may be primarily or entirely handled by a person who assists in obtaining a sample from the subject). For example, a subject will typically or always require the assistance of at least one other person when obtaining cerebrospinal fluid. During the gathering of the cerebrospinal fluid, the vessel typically will be mostly or entirely handled by a sample collection technician (rather than being handled by the subject). In another example, a subject will commonly (but not always) require the assistance of a sample collection technician when obtaining a blood or plasma sample. During the gathering of the blood or plasma, the vessel may be mostly or entirely handled by a sample collection technician. In another example, a subject may be capable of obtaining a sample from him or herself without the assistance of others, but the vessel may still be primarily handled by another. For example, a subject may go to a sample collection center, and meet with a sample collection technician, who provides the subject with a vessel for the collection of a saliva sample. The subject may then handle the vessel for a short period of time necessary to collect the saliva, and then return the vessel containing the saliva to the sample collection technician. In such a circumstance, the subject may only handle the vessel for a short period of time, and the majority of the time, the vessel is handled by a sample collection technician.

In embodiments in which a sample collection technician is involved in obtaining a sample from a subject, the vessel is already labeled with the identifier code or a related code at the time of collection of the sample. In other embodiments not in accordance with the invention, the vessel may be labeled with the identifier code or a related code after the sample is collected in the vessel. In embodiments, a portion of the identifier code or a related code is not visible to a sample collection technician. In embodiments, none of the identifier code or a related code is visible to a sample collection technician. In embodiments, the identifier code or a related code is not visible to the naked eye or is not readable by eye (e.g., the identifier code or a related code requires special illumination, or a special apparatus, or treatment of some kind, in order to be read).

In embodiments in which a sample collection technician is involved in obtaining a sample from a subject, the sample collection technician may not learn any identifying information about the subject during the sample collection process. Similarly, the subject is not required to provide any identifying information about him or herself during the sample collection process. For example, in embodiments in which a sample collection technician assists in obtaining a sample from a subject, the subject may not provide the sample collection technician with his or her first name, middle name, last name, social security number, date of birth, age, home address, work address, driver's license number, passport number, medical record number, credit card number, or biometric information. Alternatively or additionally, in embodiments in which a sample collection technician is involved in obtaining a sample from a subject, the sample collection technician may not learn which test is to be performed with the sample from the subject. For example, a sample collection technician may be aware that he or she is to collect whole blood from a subject, but not know what test will be performed with the blood.

In embodiments, a subject may select a method for providing a sample to a sample vessel. For example, at the time of ordering a laboratory test from the LACS, the subject may be informed that the sample is to be self-collected by the subject, that the sample must be collected with the aid of a sample collection technician, or that the sample may be self-collected or collected with the aid of a sample collection technician. If the sample may be self-collected or collected with the aid of a sample collection technician, the subject may be given the choice of which of these two collection methods the subject would prefer. If the subject is to self-collect the sample, the subject may be given the choice of how he or she would like to obtain the sample vessel. For example, the subject may select to have the vessel mailed to a particular location, or to pick up the vessel at a particular location. In addition, in this example, the subject may be given a choice of selecting the exact address where a vessel is to be mailed to or of a particular location where the vessel is to be picked up. Similarly, if a subject is to provide the sample with the assistance of a sample collection technician, the subject may be permitted to select a particular location and optionally, time, where the sample is to be collected.

In the event that a subject provides a sample by going to a point of service center and either i) obtaining at the point of service center a vessel to be loaded with a self-collected sample or ii) working with a sample collection technician at the point of service center to provide the sample, the subject may request the respective sample collect method without providing identifying information about him or herself. For example, at a point of service center, a subject may present his or her identifier code (which was obtained at the time of ordering the laboratory test) or a related code, and the subject may then be provided with an appropriate sample vessel for sample self-collection or sent to meet with a sample collection technician. Based on the identifier code or a related code, the point of service center can provide the subject or a sample collection technician with the correct vessel for the test(s) to be performed with the sample. During this process, it is not necessary for any person at the point of service center to know what laboratory test(s) are to be performed with a sample from the subject. Moreover, in embodiments, the identifier code or related code does not contain any information that could indicate what laboratory test(s) are to be performed with the sample to a sample collection technician or other personnel at a point of service center location. In embodiments, the identifier code or related code is not visible to, or is not readable by, one or more persons at the point of service center, so that at least some personnel at the point of service center remain without knowledge of the identity of the subject, of the tests to be performed, and/or of information in an identifier code or a related code. Thus, the confidentiality of both i) the identity of the patient and ii) the test to be performed with the sample may be preserved, even when a sample collection technician is involved in the sample collection process or laboratory technician is involved in handling a sample vessel.

Once a biological sample is obtained from the subject and loaded into a vessel, the vessel may be provided at the location of a sample processing device. A sample processing device may be located at any position relative to the site where the subject obtains a vessel or where the sample is loaded into a vessel. For example, the sample processing device may be located at a point of service location where a subject can pick up a vessel for self-collection of a sample or where a subject can have sample obtained with the aid of a sample collection technician. In another example, the sample processing device may be located at a location that is remote from locations where a subject can pick up a vessel or where sample can be obtained with the aid of a sample collection technician. In such circumstances, a subject, sample collection technician, or other party may be able to mail or otherwise send (e.g. via a courier) a vessel to the location where a sample collection device is located. In other embodiments, a subject may be able to drop off a vessel containing sample at a location configured to receive vessels, from which the vessels will be transported to the location of a sample collection device.

Once a vessel containing a sample is situated at the location of a sample processing device, sample from the vessel may be introduced into the sample processing device. In embodiments, the vessel containing the sample may be loaded into the sample processing device. In other embodiments, the vessel itself is not introduced into the sample processing device, and only the sample is introduced into the device (e.g. by wicking, pouring, suction, aspiration, pipetting, or injection of the sample into the sample processing device).

When the vessel containing the sample arrives at the location of a sample processing device, it may be directly or indirectly associated with an identifier code or a related code. When the sample from the vessel is loaded into the sample processing device, the identifier code or a related code associated with the sample vessel may be entered into the sample processing device. Any suitable method for introduction of the identifier code or related code into the sample processing device may be used. For example, if the code is a bar code, the code may be scanned by a bar code reader operatively connected to the sample processing device. In another example, if the code is an alphabetic or numeric code, the code may be scanned in by an optical reader operatively connected to the sample processing device, or typed by a technician into an alphabetic or numeric keyboard operatively connected to the sample processing device. In another example, if the identifier code is an RFID tag, the tag may be sensed by an RFID reader operatively connected to the sample processing device. Once an identifier code or related code for a sample is introduced into a sample processing device, data obtained from processing the sample may be associated by the sample processing device with the identifier code or a related code. The association of data from a sample with the identifier code or related code of the sample may be performed by a processor in or operatively coupled to the sample processing device. An identifier code or related code for a sample may be introduced into a sample processing device before, simultaneously with, or after the introduction of the sample into the sample processing device. For example, in embodiments, a code on a vessel may be scanned when the vessel is outside of the sample processing device, and then only the sample is introduced into the sample processing device. In another example, a vessel containing a sample may be introduced into a sample processing device, and once inside the device, the code on the vessel may be scanned by the sample processing device.

In embodiments, a sample or a vessel containing the sample may be loaded into a cartridge, and the cartridge may be loaded into the sample processing device. The cartridge contains reagents for performing multiple laboratory tests with the sample. In embodiments, the code from a sample vessel may be entered into the sample processing device before the sample is introduced into the device. Once the code is entered into the sample processing device, the sample processing device may provide information to a technician (e.g. by a screen, audio, print-out, etc.) about what type of cartridge out of a plurality of available cartridge types to load the sample into. For example, 5 different cartridge types may be available at the sample processing device. In this example, if a laboratory technician enters the code from the sample vessel into the sample processing device, the device may display on a screen "Cartridge Type 4". The laboratory technician may then load the sample into a "Type 4" cartridge, and then load the cartridge containing the sample into the sample processing device. According to the invention, a cartridge contains reagents for performing 2, 3, 4, 5, 6, 7, 8, 9, 10, 15, 20 or more different laboratory tests with a sample from a subject. In some situations, if a sample is loaded into or used with a cartridge which contains reagents for performing two or more different laboratory tests, not all of the laboratory tests will be performed on a sample (e.g. based on a protocol for processing the sample, which is based on the tests requested by the subject). For example, a cartridge may contain reagents for performing ten different laboratory tests, but a subject may have only requested three of these tests to be performed. In embodiments, only these three tests will be performed (rather than all ten available tests on the cartridge). In situations in which a cartridge contains reagents for performing two or more tests and less than the number of available tests is performed with the sample, the confidentiality as to the subject's test may further increase. For instance, even if a laboratory technician is aware that a cartridge contains reagents for performing 10 different tests, if less than the 10 tests are performed with the sample, than the technician may not know which of the 10 tests were performed with the sample. In embodiments, a cartridge may contain reagents for performing at least n different laboratory tests, and no more than n-1 tests are performed with the sample. *n* may be, for example, 2, 3, 4, 5, 6, 7, 8, 9, 10, 15, 20, 25, 50, 100, or more. Thus, for example, even if a cartridge contains reagents for a certain test (e.g. HIV), if the cartridge contain reagents for multiple tests (e.g. HIV, influenza, and Staphylococcus), a laboratory technician won't know, for example, if a sample using the cartridge is being tested for HIV.

A sample in a sample processing device may be processed according to a protocol. The protocol may include one or more steps for processing the sample to perform the laboratory test requested by the subject. A protocol may be executed by a processor of the sample processing device, which may control the operations of one or more components of the sample processing device. A sample processing device may be any device capable of processing a biological sample to yield data for obtaining laboratory results. Description and disclosure of sample processing devices which may be used with systems and methods provided herein may be found, for example, in U.S. Patent 8,088,593; U.S. Patent 8,380,541; U.S. Pat. App. Ser. No. 13/769,798, filed February 18, 2013; U.S. Pat. App. Ser. No. 13/769,779, filed February 18, 2013; U.S. Pat. App. Ser. No. 13/244,947 filed Sept. 26, 2011; PCT/US2012/57155, filed September 25, 2012; U.S. Application Serial No. 13/244,946, filed September 26, 2011; U.S. Patent Application 13/244,949, filed September 26, 2011; and U.S. Application Serial No. 61/673,245, filed September 26, 2011. In embodiments, sample processing devices for use with systems and method provided herein may rapidly process a biological sample from a subject, in order to be able to rapidly provide test results to a subject. For example, systems and methods provided herein may be able to process a sample and have results available for a subject within 24 hours, 16 hours, 12 hours, 8 hours, 4 hours, 3 hours, 2 hours, 1 hour, 45 minutes, 30 minutes, 20 minutes, 15 minutes, 10 minutes, 5 minutes, 4 minutes, 3 minutes, 2 minutes, 1 minute, 45 seconds, 30 seconds, 15 seconds, 10 seconds, 5 seconds, 2 seconds, or 1 second of the biological sample being provided to a sample processing device. In embodiments, with systems and methods provided herein, a biological sample may be rapidly processed with a process involving nucleic acid amplification, wherein the nucleic acid amplification is performed according to a method provided in U.S. Provisional Patent Application No. 61/800,340, U.S. Provisional Patent Application No. 61/800,606, or U.S. Provisional Patent Application No. 61/802,241, each filed on March 15, 2013.

A protocol for processing a sample in a sample processing device may be provided from various different sources. For example, in embodiments, one or more protocols may be locally stored in a data storage unit in the sample processing device. Information in the identifier code or a related code associated with the sample vessel may instruct the sample processing device as to the protocol that is to be used with the sample. In another example, one or more protocols may be stored in the LACS. When the sample processing device obtains an identifier code or related code for a sample, the sample processing device may send the code to the LACS. In response to receiving the code, the LACS may send a protocol to the sample processing device. In another example, in response to receiving the code, the LACS may send instruction information to the sample processing device, and then a processor on the sample processing device may use the instruction information to select a protocol already stored in a data storage unit of the sample processing to use for processing the sample. In another example, a LACS may generate a protocol upon the receipt of an identifier code or related code, and then send the protocol to the sample processing device for processing the sample.

The sample obtained from the subject may be processed on the device according to a protocol. Processing may involve any assay, reagent, or sample processing step as described elsewhere herein or in any patent or patent application referenced elsewhere herein, and may include, for example, sonication, centrifugation, dilution, pipette-based dilution or transfer steps, immunoassay steps, or nucleic acid amplification steps. Processing may involve one or more steps of a laboratory test requested by a subject. In embodiments, a sample processing device may perform some or all of the steps of a laboratory test which involve physically processing a biological sample (e.g. diluting a sample, mixing a sample with one or more reagents, or obtaining a signal from a reaction performed with the sample and one or more reagents).

Based on the processing of the sample, the sample processing device generates information regarding the sample, which it sends to the LACS. In some embodiments, the sample processing device may process the sample according to the test to be performed on the sample, and generate the final laboratory test results, which are sent to the LACS. The final laboratory test results may be in any format as appropriate for the test (e.g. a positive or negative result in the case of a test such as pregnancy status, or a result which gives a relative level of an analyte in the case of a test such as for illegal drug concentration). In other embodiments, the sample processing device may perform one or more processing steps with the sample, and generate raw data relating to the processing steps. The sample processing device may then send the raw data to the LACS, which may further process the data to complete the laboratory test and to generate the finalized test results.

Once the LACS obtains the results for the test with the subject's sample (e.g. by receiving the results from the sample processing device or by generating the results with data from the sample processing device), the subject may access the LACS to obtain the results of the test(s) he or she ordered. In embodiments, the subject may engage with a personal computing device and request the test results from the LACS. For example, a subject may log-on to a web-site associated with the LACS, or to a web-site which provides access to the LACS (e.g., which provides secure access to the LACS), and may receive test results by entering, or otherwise providing, the proper identifier code or related code. The personal computing device may the same computing device as was used to request the test, or a different computing device.

In embodiments, during the process of ordering the laboratory test, the LACS may provide the option of permitting a subject to require that the laboratory test results only be accessible by the same personal computing device as was used to request the laboratory test. By requiring that the same personal computing device be used to order the laboratory test and to view the test results, unauthorized access to test results may be further restricted.

In embodiments, the subject may be made aware that the laboratory results are ready to access by a notification from the LACS. For example, during the process of ordering a laboratory test, the subject may select a method for being notified when the results are ready. Such methods may include, for example, by text, e-mail, or postal mail. In order to preserve the confidentiality of the subject's identity, the subject may, for example, set up an e-mail address with a false name or which otherwise cannot be readily connected to his or her true identity. In other embodiments, a subject may be aware that laboratory test results are ready to access based on an amount of time elapsed after the subject provided the biological sample in a vessel and provided the vessel at a selected location. For example, during the ordering process for a test, a subject may be informed that the test results will be available 24 hours after providing a vessel containing the biological sample at a point of service location. In other embodiments, in order to determine when the laboratory results will be available, the subject may provide his or her identifier code or a related code to the LACS, and the subject may receive information indicating if the results are available, and if not, at what time the results will be available.

In order to request the test results, the subject provides the identifier code or a related code to the LACS. In some embodiments, only the actual identifier code (and not a code related to the identifier code) may be used to access the test results. As discussed elsewhere herein, in embodiments, security of the test results may be increased by using a code related to the identifier code to label a sample vessel, and using the actual identifier code to access the test results.

In embodiments, in order to access the test results, the subject may be required to answer one or more questions. These questions may be in addition to or separate from a requirement to provide a valid identifier code or related code. The questions may be of the type that subject is will generally know but that others generally may not know. Questions may include, for example: on what date did you provide a biological sample?; what type of biological sample did you provide?; on what date did you order the test?; where did you provide the biological sample?, etc. Requiring a subject to answer one or more questions, in addition to providing an identifier code or related code, may further increase the security of systems and methods provided herein, and may further help prevent unauthorized access to laboratory test results.

Upon receipt of a valid identifier code or related code, the LACS provides the test results to the subject on the personal computing device. The results may be accompanied by explanatory information, guidelines for next steps in view of the test results, or other information. In embodiments, a subject may request or access test results in other ways. For example, a subject may use a personal computing device to request his or her test results, but request that the results be sent to a point of service center (e.g. by mail or to a computer terminal), where the subject can access the test results by providing an identifier code or related code to a laboratory technician or instrument that can display, print out, or otherwise provide the results. In another example, at the time of ordering a laboratory test, a subject may request that when available, the test results be sent to a point of service center, where they can be accessed by providing an identifier code or related code to a laboratory technician or instrument that can display, print out, or otherwise provide the results. When a subject selects a point of service center, the subject may have the option of selecting the location (e.g. a specific location or a geographic region) where the subject would like to access the test results.

In embodiments, a subject may receive counseling, care, or advice or the like in response to the test results, such as from a health care professional (e.g. a medical doctor, nurse, or pharmacist). In embodiments, a subject may have the option of asking questions related to his or her test results. A subject may ask questions about his or her test results without providing identifying information about him or herself. For example, a subject may anonymously send a text or e-mail based question via a personal computing device to a health care professional associated with the LACS. In another embodiment, a subject may call a health care professional associated with a LACS or laboratory administrator. In embodiments, a subject's voice may be altered by a digital or analog device, in order to further preserve the confidentiality of the subject.

After the LACS has provided the laboratory test results to the subject, the LACS may continue to store the results, or may delete the results. The LACS may store the results, for example, to permit a subject to access the results and any related information at a later time. In embodiments, the LACS may store the results, but in a manner that is accessible only upon the fulfillment of special conditions (e.g., only upon specific approval of the subject, or only with use of the identification code or other code, or only for specific uses, or only to be provided to specific persons or entities). In embodiments, the LACS may erase the results, for example, to increase the security of the test results. In embodiments, when a subject orders a laboratory test, the subject may have the option of choosing whether his or her laboratory test results are to be saved or erased by the LACS, or a time period for which the results are to be saved, after which the results are to be erased by the LACS.

In embodiments, systems and methods provided herein may include features for permitting a subject to pay for an ordered laboratory test. A subject may a pay for a laboratory test in various different ways. In embodiments, with systems and methods provide herein, a subject may pay for a laboratory test in a way that does not involve disclosing information relating to the identity of the subject during the payment. In one embodiment, a subject may pay for a laboratory test by using a pre-paid debit card, credit card, gift card, voucher, or the like (collectively referred to herein as a "pre-paid" card). To use a pre-paid card, in embodiments, the subject may first put monetary value on the card. To put value on a pre-paid card, the subject may purchase a new pre-paid card or add value to an existing pre-paid card at any suitable location (e.g. in a retail location, on a website which sells pre-paid cards). In embodiments, a subject may put value on a pre-paid card using cash, so that the pre-paid card is not linked to any information relating to the identity of the subject. In other embodiments, a subject may put value on a pre-paid card using a personal credit or debit card, which may be linked to information relating to the identity of the subject. Nonetheless, even if a personal credit or debit card is used to put value on a pre-paid card, the resulting pre-paid card may still not be closely tied to identifying information relating the subject, and such a pre-paid card may provide a satisfactory level of identity protection for a subject. In other embodiments, an organization (e.g. school, company, community health organization, health insurance company, nonprofit organization, governmental organization, etc.) may purchase one or more laboratory tests from an entity providing a system or method provided herein. In return for the purchase, the organization may receive vouchers or pre-paid cards for laboratory tests, which the organization may then distribute to subjects. These vouchers or pre-paid cards may not be linked to a particular subject (e.g. no record is kept of which subject receives which voucher or pre-paid card, and possibly, no record is kept of which subjects receives any vouchers or pre-paid cards at all). The subjects may they use the vouchers or pre-paid cards to pay for laboratory tests.

In embodiments in which a subject pays for a laboratory test with a pre-paid card, the subject may pay on a personal computing device (e.g. by typing the card information into an interface with a LACS on a personal computing device, or by swiping the card on a card reader in or associated with the personal computing device). In embodiments, a personal computing device may be a computer terminal, kiosk, or the like in a retail location. In embodiments, a subject may use a pre-paid card when buying a kit at a retail location (described further below). In other embodiments, a subject may pay for a laboratory test using a personal credit or debit card. A subject may pay for a laboratory test with a personal credit or debit card in any of the situations disclosed herein wherein the subject may pay with a pre-paid card. Although use of a personal credit or debit card to pay for a laboratory test may involve disclosure of some information relating to the identity of the subject, some subjects may find this to be acceptable.

In embodiments in which the personal computing device is located at a retail location, the personal computing device may be configured to receive one or more of pre-paid cards, personal credit or debit cards, or cash. For example, a personal computing device may be operably connected to a credit card reader, it may contain an interface for permitting a user to type a card numeric or alphabetic code into it, or it may contain an automated cash receiving device.

In other embodiments, a subject may pay for a laboratory test by purchasing at a retail location a kit for a laboratory test (which may contain, for example, a collection vessel) or voucher for a laboratory test. The price of the kit or voucher may include the price for the laboratory test. In such embodiments, a subject may pay for the test with a pre-paid card, a personal credit or debit card, or cash.

With systems and methods provided herein, a subject may pay for a laboratory test at various points in the testing process. For example, the subject may provide payment at the time of ordering the test. In another example, the subject may be required to provide payment before the subject can collect or receive a sample vessel. In another example, the subject may be required to provide payment before the LACS will provide the results of the laboratory test to the subject. Operators of a system or method described herein may select any desired point in a method provided herein by which to require a subject to pay for a test.

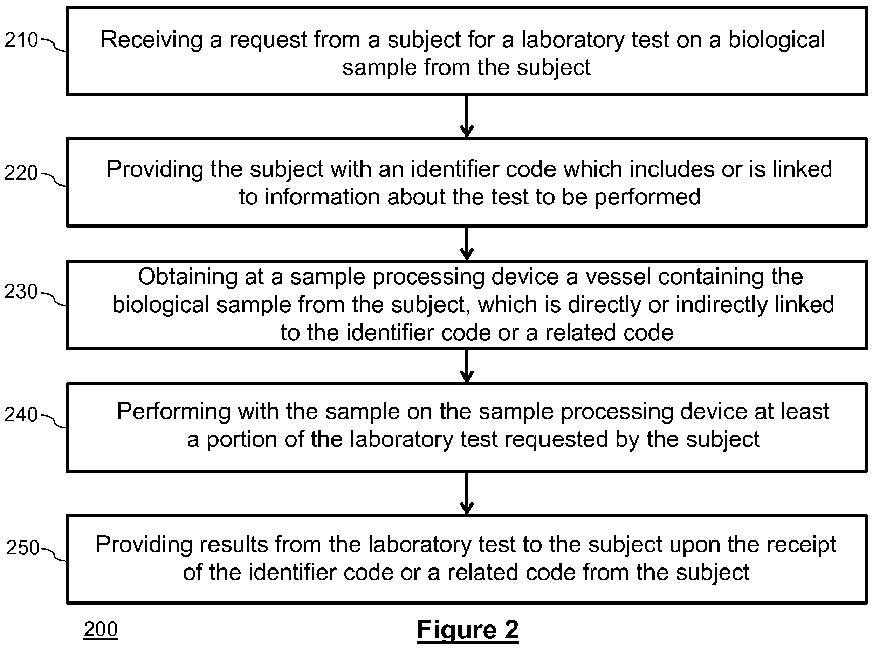

In another example, embodiments of systems and methods provided herein may be described with reference to Figure 2, which provides steps of an exemplary method provided herein 200. A laboratory test administrator may receive a request from a subject for a laboratory test, which is to be performed with a biological sample from the subject 210. The laboratory test administrator may be a laboratory associated computer system (LACS) or other entity or computing system. In embodiments, a laboratory test administrator may involve human participants. For example, the laboratory test administrator may include human technicians which can receive requests for a laboratory test from a subject. For example, a subject may call a phone number associated with the laboratory test administrator and speak with a technician to order the test and receive follow up information from the laboratory test administrator. In embodiments, in this or other steps of methods provided herein, a subject may access the laboratory test administrator, for example, by a personal computing device, by phone, or in person. The subject is not required to provide any information relating to his or her identity in order to request a laboratory test. The subject may request one or more individual tests or panels of tests. The tests may be selected from a menu of available tests, provided by the test administrator. In response to the subject's request, the laboratory test administrator may provide the subject with an identifier code which contains or is linked to information about the test to be performed with the biological sample from the subject 220. The identifier code may have any of the characteristics or features of identifier codes described elsewhere herein. Next, a vessel containing a biological sample from the subject may be provided at a sample processing device 230. Sample may be loaded into the vessel and the vessel may be transported to a sample processing device through any suitable process as described elsewhere herein. The vessel may be directly or indirectly labeled with the identifier code provided to the subject, or a related code. Using sample from the vessel, the sample processing device may perform at least a portion of the laboratory test ordered by the subject 240. The sample processing device may have any of the features described elsewhere herein. In certain embodiments, the sample processing device may process the sample, analyze data obtained from processing the sample, and determine the results of the laboratory test. In other embodiments, the sample processing device may process the sample to generate raw data, and then provide the raw data to another entity (e.g. the laboratory test administrator), which then generates the laboratory test result. After the processing of the sample by the sample processing device, the laboratory test administrator may obtain the laboratory test results. The laboratory test administrator may obtain the test results by calculating the results itself (e.g. with data generated by the sample processing device), or the laboratory test administrator may receive the test results from another entity (e.g. from a sample processing device which both generates and analyzes data). Once the laboratory test administrator has the subject's laboratory test results, the subject may request the test results from the laboratory test administrator. In order to receive the test results, the subject may be required to provide the identifier code (which he or she previously received from the laboratory test administrator) or a related code to the laboratory test administrator 250. Upon receipt of the identifier code or related code, the laboratory test administrator may provide the test results to the subject. Using this method, a subject may request, have performed, and receive results of a laboratory test, without having to provide any information relating to his or her identity.

In embodiments, some or all of the steps provided herein may be performed over a computer network. For instance, a subject may use a personal computing device to order a laboratory test from a laboratory test administrator, where the personal computing device and the laboratory test administrator are operably connected via a computer network. In embodiments, the laboratory test administrator may be a computerized system, such as a laboratory associated computer system described elsewhere herein. In embodiments, the laboratory test administrator functions are carried out by a mixture of computerized systems and human activity. Typically, the biological sample from the subject is physically transported to a sample processing device (or vice versa) for analysis.

In embodiments, two or all three of a personal computing device, LACS and sample processing device may be operatively coupled without a network intermediary (e.g. a personal computing device and a LACS may be directly linked as part of the same device or a personal computing device, LACS, and sample processing device may be directly linked as part of the same device). Also provided herein are systems containing devices which physically integrate two or all three of a personal computing device, LACS and sample processing device. Such devices may have additional features, such as modules to accept payment (e.g. credit card readers, keypads, automated cash receivers) and modules for dispensing or receiving sample vessels (e.g. motorized levers, coils or belts which can transport vessels). Such devices may be provided in publically accessible locations, so that subjects can easily access the devices. Methods provided herein may be performed on such devices. Also, in embodiments, a subject may use a device which integrates a personal computing device with one or both of a LACS or a sample processing device for at least one step of a method provided herein, and then uses a different personal computing device for another step of a method provided herein. For example, in embodiments, a subject may order a laboratory test on a personal computing device integrated with a LACS which is available in a drug store, and then the subject may use a personal computer in his or her home to access to the laboratory test results.

In embodiments, components of systems provided herein may be physically separate from each other. For example, a personal computing device, LACS, and sample processing device may each be separated from each other by at least 1, 10, 100, or 1000 meters. In embodiments, components of systems provided herein may be in different rooms or buildings. In other embodiments, one or more components of systems provided herein may be provided together in a same location. For example, in embodiments, one or more components of systems provided herein may be within a common housing. In an example, a LACS may be physically coupled to or in the same room or building as a sample processing device. In another example, a LACS may be physically coupled to or in the same room or building as a personal computing device. A personal computing device / LACS combination, for example, may be in a public location where a subject may access the personal computing device to order a laboratory test. In embodiments, a personal computing device, LACS, and sample handling device may be physically coupled or in the same room or building.

In embodiments, provided herein are systems for providing laboratory tests. The systems may have any of the features provided in Figures 1 or 2 or described elsewhere herein. In embodiments, a system may include one, two, or all three of a LACS, a sample processing device, and a personal computing device.

In embodiments, provided herein are non-transitory computer-readable media comprising machine-executable code for implementing methods provided herein. Non-transitory computer readable media comprising machine executable code can contain any of the features described elsewhere herein. In embodiments, the medium may be in a server, a hard drive, or a portable data storage unit.

In embodiments, with systems and methods provided herein, a subject may create an account which is not associated with information relating to the identity of the subject, or which is only associated with a limited amount of information relating to the identity of a subject. For example, a subject may create an account which is associated with no more than 3, 2, 1, or 0 of any one or more of the subject's first name, middle name, last name, social security number, date of birth, age, home address, work address, driver's license number, passport number, medical record number, and credit card number. The account may be provided by a laboratory administrator (e.g. a LACS) and may be accessible on a personal computing device. With the account, the subject may store health related information, including laboratory test results. Using such an account, a subject may, for example, track test results over time. To create an account, a user may, for example, receive an identifier code as described herein. The subject can then use the identifier code as a code to access an account associated with the identifier code. Once an account is established a subject can, for example, use the identifier code or a related to code for subsequent tests to be associated with the account. In embodiments, a subject may be able to select a password or user account number or the like without providing information relating to his or her identity. Accordingly, systems and methods provided herein may be used not only for providing a one-time test result to a subject, but also for creating accounts and for storing multiple laboratory test results and providing result of multiple laboratory test results to subjects over a period of time.

This application claims the benefit of, and priority to, U.S. Provisional Patent Application No. 61/895,239, filed October 24, 2013.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. For example, a feature of one embodiment may be combined with a feature of another embodiment, whether such combination is described herein or not. It should also be understood that while the invention provided herein has been described herein using a limited number of terms and phrases for purposes of expediency, the invention could also be described using other terms and phrases not provided herein which also accurately describe the invention.

It should be understood that as used in the description herein and throughout the claims that follow, the meaning of "a," "an," and "the" includes plural reference unless the context clearly dictates otherwise. For example, a reference to "an assay" may refer to a single assay or multiple assays. Also, as used in the description herein and throughout the claims that follow, the meaning of "in" includes "in" and "on" unless the context clearly dictates otherwise. The appended claims are not to be interpreted as including means-plus-function limitations, unless such a limitation is explicitly recited in a given claim using the phrase "means for." As used in the description herein and through the claims that follow, a first object described as containing "at least a portion" of a second object may contain the full amount of / the complete second object.

As used in the description herein and throughout the claims that follow, the terms "comprise", "include", and "contain" and related tenses are inclusive and open-ended, and do not exclude additional, unrecited elements or method steps. Also, the presence of broadening words and phrases such as "one or more," "at least," "but not limited to" or other like phrases in some instances shall not be read to mean that the narrower case is intended or required in instances where such broadening phrases may be absent. Finally, as used in the description herein and throughout the claims that follow, the meaning of "or" includes both the conjunctive and disjunctive unless the context expressly dictates otherwise. Thus, the term "or" includes "and/or" unless the context expressly dictates otherwise.

This document contains material subject to copyright protection. The copyright owner (Applicant herein) has no objection to facsimile reproduction by anyone of the patent documents or the patent disclosure, as they appear in the US Patent and Trademark Office patent file or records, but otherwise reserves all copyright rights whatsoever. The following notice shall apply: Copyright 2013-14 Theranos, Inc.

## Claims

1. A method for providing laboratory test results to a subject, comprising:
A) receiving by a laboratory associated computer system (LACS) (125) a request from a subject (110) for a laboratory test, wherein the laboratory test is to be performed with a biological sample from the subject, and wherein the request from the subject for a laboratory test is associated with no information relating to the identity of the subject, or no more than three pieces of such information;
B) providing by the LACS (125) an identifier code to the subject in response to the request, wherein the identifier code comprises or is linked to information regarding the laboratory test to be performed with the sample from the subject;
providing a vessel (130) to the subject (110) for sample collection, wherein the said vessel (130) is provided to the user with an identifier having the identifier code or a related code already on the said vessel when the vessel is received by the user;
C) obtaining at a sample processing device (135) said vessel (130) containing the biological sample from the subject, wherein the said vessel is associated with the identifier code or a related code;
D) processing the biological sample on the sample processing device (135) according to the laboratory test requested by the subject (110), wherein the sample processing device (135) is in communication with the LACS and wherein information related to the sample is transmitted to the LACS (125); wherein the said vessel (130) is handled by a laboratory technician during at least one of step C) or D) and wherein the sample processing device (135) uses a cartridge that contains reagents to multiple tests, so that a laboratory technician does not know which subset of tests were actually performed, and
E) providing by the LACS (125) results from the laboratory test to the subject (110), wherein the results from the laboratory test are provided to the subject after the subject provides the identifier code or a related code to the LACS (125).

2. The method of claim 1, wherein the LACS (125) is accessible via a network (120), and wherein the subject (110) enters the request for the laboratory test on a personal computing device (115) operably connected to the LACS (125) via said network (120).

3. The method of claims 1 or 2, wherein the laboratory test is for an infectious disease, a genetic disease or condition, pregnancy status, or a sexually-transmitted disease.

4. The method of any of claims 1, 2, or 3, wherein in step A) the request from the subject (110) for a laboratory test is associated with said no more than three pieces of such information selected from the group consisting of the subject's: first name, middle name, last name, social security number, date of birth, age, home address, work address, driver's license number, passport number, medical record number, or credit card number.

5. The method of any of claims 1-4, wherein the cartridge is labelled with a non-assay specific identifier so that the laboratory technician does not know what assay is being performed.

6. The method of any of claims 1-5, further comprising prior to step D), inserting the vessel (130) containing the biological sample into a cartridge containing reagents for performing two or more different laboratory tests.

7. The method of claim 6, wherein less than the total number of laboratory tests which the cartridge contains reagents for performing are performed with the biological sample.

8. The method of any of claims 1-7, wherein the information transmitted to the LACS (125) comprises raw data from the sample processing device (135).

9. The method of any of claims 1-8, further comprising receiving by the LACS (125) a payment by the subject for the laboratory test.

10. The method of any of claims 1-9, wherein the LACS (125) does not store any information relating to the identity of the subject during the course of the method.

11. A system for providing laboratory test results to a subject (110), comprising:
A) a laboratory associated computer system (LACS) (125), wherein the LACS is configured to: i) receive a request from a subject (110) for a laboratory test, wherein the request is associated with no or not more than three pieces of information relating to the identity of subject and wherein the laboratory test is to be performed with a biological sample from the subject (110); ii) provide an identifier code to a subject (110) in response to the request, wherein the identifier code comprises or is linked to information regarding the laboratory test to be performed with the sample from the subject (110); and iii) provide results from the laboratory test to the subject (110), after the performance of the laboratory test and upon receipt by the LACS (125) of the identifier code or a related code from the subject (110);
a vessel (130) for sample collection, wherein said vessel (130) has an identifier having the identifier code or a related code already on the said vessel when the said vessel is received by the subject (110) and prior to sample being added to the said vessel (130); and
B) a sample processing device (135), wherein the sample processing device (135) is in communication with the LACS (125) and wherein the sample processing device (135) is configured to: i) process the biological sample according to the laboratory test requested by the subject (110), upon the receipt at the location of the sample processing device (135) of said vessel (130) containing the biological sample from the subject (110), wherein the said vessel (130) is associated with the identifier code or a related code, and ii) transmit information related to the sample to the LACS (125), wherein the sample processing device (135) uses a cartridge that contains reagents to multiple tests, so that a laboratory technician handling the vessel (130) does not know which subset of tests were actually performed.

12. The system of claim 11, wherein the LACS (125) and the sample processing device (135) are in separate locations.

## Patentansprüche

1. Verfahren für die Bereitstellung von Labortestergebnissen an ein Subjekt, umfassend:
A) Empfangen über ein mit einem Labor verbundenes Computersystem (LACS) (125) einer Anfrage eines Subjekts (110) zu einem Labortest, wobei der Labortest mit einer biologischen Probe von dem Subjekt ausgeführt werden muss und wobei die Anfrage von dem Subjekt zu einem Labortest mit keinerlei Information zur Identität des Subjekts verbunden ist, oder mit nicht mehr als drei Teilen solcher Informationen;
B) Bereitstellen durch das LACS (125) eines Identifikationscodes an das Subjekt als Antwort auf die Anfrage, wobei der Identifikationscode Informationen zu dem Labortest, der mit der Probe von dem Subjekt ausgeführt wird, umfasst oder damit verbunden ist;
Bereitstellen eines Gefäßes (130) an das Subjekt (110) für die Probenentnahme, wobei das Gefäß (130) dem Benutzer mit einem Identifikator bereitgestellt wird, der den Identifikationscode trägt oder einen zugehörigen Code, der bereits auf dem Gefäß angebracht ist, wenn das Gefäß vom Benutzer übernommen wird;
C) Entgegennehmen in einer Probenentnahmevorrichtung (135) dieses Gefäß (130), das die biologische Probe von dem Subjekt enthält, wobei dieses Gefäß mit dem Identifikationscode oder dem zugehörigen Code verbunden ist;
D) Verarbeiten der biologischen Probe in der Probenverarbeitungsvorrichtung (135) gemäß dem vom Subjekt (110) angefragten Labortest, wobei die Probenverarbeitungsvorrichtung (135) in Verbindung mit dem LACS steht und wobei die mit der Probe verbundenen Informationen an das LACS (125) übermittelt werden; wobei dieses Gefäß (130) von einem Labortechniker in mindestens entweder Schritt C) oder D) bearbeitet wird und wobei die Probenverarbeitungsvorrichtung (135) eine Kartusche verwendet, die Reagenzien für mehrere Tests enthält, so dass ein Labortechniker nicht weiß, welche Untergruppe der Tests tatsächlich ausgeführt wird, und
E) Bereitstellen durch das LACS (125) von Ergebnissen aus dem Labortest an das Subjekt (110), wobei die Ergebnisse aus dem Labortest dem Subjekt bereitgestellt werden, sobald das Subjekt den Identifikationscode oder einen zugehörigen Code dem LACS (125) vorlegt.

2. Verfahren nach Anspruch 1, wobei das LACS (125) über ein Netzwerk (120) erreichbar ist und wobei das Subjekt (110) die Anfrage zu dem Labortest in ein Personalcomputer-Gerät (115) eingibt, das betriebsbereit mit dem LACS (125) über dieses Netzwerk (120) verbunden ist.

3. Verfahren nach Anspruch 1 oder 2, wobei der Labortest für eine Infektionskrankheit, eine Erbkrankheit oder ein genetisches Leiden, einen Schwangerschafts-Status oder eine sexuell übertragbare Erkrankung eingesetzt wird.

4. Verfahren nach einem der Ansprüche 1, 2 oder 3, wobei in Schritt A) die Anfrage von dem Subjekt (110) zu einem Labortest mit den nicht mehr als drei Teilen solcher Informationen verbunden ist, ausgewählt aus der Gruppe bestehend aus folgenden Angaben zum Subjekt: Vorname, Mittelname, Nachname, Sozialversicherungsnummer, Geburtsdatum, Alter, Wohnadresse, Arbeitsadresse, Führerscheinnummer, Reisepassnummer, Krankenaktennummer oder Kreditkartennummer.

5. Verfahren nach einem der Ansprüche 1-4, wobei die Kartusche mit einem nicht untersuchungsspezifischen Identifikator etikettiert ist, so dass der Labortechniker nicht weiß, welche Untersuchung ausgeführt wird.

6. Verfahren nach einem der Ansprüche 1-5, ferner umfassend vor Schritt D), Einsetzen des Gefäßes (130), das die biologische Probe enthält, in eine Reagenzien enthaltende Kartusche für die Durchführung von zwei oder mehr verschiedenen Labortests.

7. Verfahren nach Anspruch 6, wobei weniger als die Gesamtzahl der Labortests, für die die Kartusche Reagenzien für eine Durchführung des Tests enthält, mit der biologischen Probe durchgeführt werden.

8. Verfahren nach einem der Ansprüche 1-7, wobei die an das LACS (125) übermittelten Informationen Rohdaten aus der Probenverarbeitungsvorrichtung (135) umfassen.

9. Verfahren nach einem der Ansprüche 1-8, ferner umfassend das Empfangen durch das LACS (125) einer Zahlung von dem Subjekt für den Labortest.

10. Verfahren nach einem der Ansprüche 1-9, wobei das LACS (125) keine Informationen bezüglich der Identität des Subjekts im Verlauf des Verfahrens speichert.

11. System für die Bereitstellung von Labortestergebnissen an ein Subjekt (110), umfassend:
A) Ein mit einem Labor verbundenes Computersystem (LACS) (125), wobei das LACS für Folgendes konfiguriert ist: i) Empfangen einer Anfrage eines Subjekts (110) zu einem Labortest, wobei die Anfrage mit keinen oder nicht mehr als drei Teilen von Informationen zur Identität des Subjekts verbunden ist und wobei der Labortest mit einer biologischen Probe von dem Subjekt (110) durchgeführt werden muss; ii) Bereitstellen eines Identifikationscodes an ein Subjekt (110) als Antwort auf die Anfrage, wobei der Identifikationscode Informationen zu dem Labortest umfasst oder damit verbunden ist, der mit der Probe von dem Subjekt (110) durchgeführt werden soll; und iii) Bereitstellen von Ergebnissen aus dem Labortest an das Subjekt (110), nach der Durchführung des Labortests und bei Empfang durch das LACS (125) des Identifikationscodes oder des zugehörigen Codes von dem Subjekt (110);
ein Gefäß (130) für die Probenentnahme, wobei dieses Gefäß (130) einen Identifikator mit dem Identifikationscode oder einem zugehörigen Code trägt, der bereits am Gefäß angebracht war, als es von dem Subjekt (110) in Empfang genommen wurde und bevor die Probe in das Gefäß (130) gegeben wurde; und
B) eine Probenverarbeitungsvorrichtung (135), wobei die Probenverarbeitungsvorrichtung (135) in Verbindung mit dem LACS (125) steht und wobei die Probenverarbeitungsvorrichtung (135) für Folgendes konfiguriert ist: i) Verarbeiten der biologischen Probe gemäß dem von dem Subjekt (110) angefragten Labortest, bei Empfang an dem Standort der Probenverarbeitungsvorrichtung (135) dieses Gefäßes (130), das die biologische Probe von dem Subjekt (110) enthält, wobei dieses Gefäß (130) mit dem Identifikationscode oder dem zugehörigen Code verbunden ist, und ii) Übertragen der Informationen zu der Probe an das LACS (125), wobei die Probenverarbeitungsvorrichtung (135) eine Kartusche verwendet, die Reagenzien für mehrere Tests enthält, so dass ein Labortechniker, der das Gefäß (130) bearbeitet, nicht weiß, welche Untergruppe der Tests tatsächlich ausgeführt wird.

12. System nach Anspruch 11, wobei sich das LACS (125) und die Probenverarbeitungsvorrichtung (135) an verschiedenen Standorten befinden.

## Revendications

1. Procédé destiné à la fourniture à un sujet des résultats de test de laboratoire, comprenant :
A) la réception par un système informatique associé à un laboratoire (LACS) (125) d'une demande provenant d'un sujet (110) pour un test de laboratoire, dans lequel le test de laboratoire doit être réalisé avec un échantillon biologique provenant du sujet et dans lequel la demande provenant du sujet pour un test de laboratoire n'est associée à aucune information se rapportant à l'identité du sujet, ou à pas plus de trois éléments de ces informations ;
B) la fourniture au sujet par le LACS (125) d'un code d'identification en réponse à la demande, dans lequel le code d'identification comprend ou est lié à des informations concernant le test de laboratoire à réaliser avec l'échantillon provenant du sujet ;
la fourniture au sujet (110) d'un récipient (130) destiné au prélèvement d'échantillon, dans lequel ledit récipient (130) est fourni à l'utilisateur avec un identifiant présentant le code d'identification ou un code associé déjà sur ledit récipient lorsque le récipient est reçu par l'utilisateur ;
C) l'obtention au niveau d'un dispositif de traitement d'échantillon (135) dudit récipient (130) contenant l'échantillon biologique provenant du sujet, dans lequel ledit récipient est associé au code d'identification ou à un code associé ;
D) le traitement de l'échantillon biologique sur le dispositif de traitement d'échantillon (135) selon le test de laboratoire demandé par le sujet (110), dans lequel le dispositif de traitement d'échantillon (135) est en communication avec le LACS et dans lequel les informations associées à l'échantillon sont transmises au LACS (125) ; dans lequel ledit récipient (130) est manipulé par un technicien de laboratoire pendant au moins une des étapes C) ou D) et dans lequel le dispositif de traitement d'échantillon (135) utilise une cartouche qui contient des réactifs pour de multiples tests, de manière à ce qu'un technicien de laboratoire ne sache pas quel sous-ensemble de tests a été réellement réalisé, et
E) la fourniture au sujet par le LACS (125) des résultats provenant du test de laboratoire (110), dans lequel les résultats provenant du test de laboratoire sont fournis au sujet après que le sujet fournisse le code d'identification ou un code associé au LACS (125).

2. Procédé selon la revendication 1, dans lequel le LACS (125) est accessible par l'intermédiaire d'un réseau (120) et dans lequel le sujet (110) entre la demande de test de laboratoire sur un dispositif informatique personnel (115) connecté de manière opérationnelle au LACS (125) par l'intermédiaire dudit réseau (120).

3. Procédé selon les revendications 1 ou 2, dans lequel le test de laboratoire concerne une maladie infectieuse, une maladie ou un état génétique, un état de grossesse ou une maladie sexuellement transmissible.

4. Procédé selon l'une quelconque des revendications 1, 2 ou 3, dans lequel, à l'étape A), la demande provenant du sujet (110) pour un test de laboratoire est associée auxdits pas plus de trois éléments d'informations de ce type choisis dans le groupe constitué par : le prénom, le second prénom, le nom, le numéro de sécurité sociale, la date de naissance, l'âge, l'adresse du domicile, l'adresse du travail, le numéro de permis de conduire, le numéro de passeport, le numéro de dossier médical ou le numéro de carte de crédit du sujet.

5. Procédé selon l'une quelconque des revendications 1 à 4, dans lequel la cartouche est étiquetée avec un identifiant non spécifique à un test, de manière à ce que le technicien de laboratoire ne sache pas quel test est en cours de réalisation.

6. Procédé selon l'une quelconque des revendications 1 à 5, comprenant en outre, avant l'étape D), l'insertion du récipient (130) contenant l'échantillon biologique dans une cartouche contenant des réactifs destinés à la réalisation de deux ou plusieurs tests de laboratoire différents.

7. Procédé selon la revendication 6, dans lequel moins que le nombre total de tests de laboratoire que la cartouche contient des réactifs destinés à la réalisation sont réalisés avec l'échantillon biologique.

8. Procédé selon l'une quelconque des revendications 1 à 7, dans lequel les informations transmises au LACS (125) comprennent des données brutes provenant du dispositif de traitement d'échantillon (135).

9. Procédé selon l'une quelconque des revendications 1 à 8, comprenant en outre la réception par le LACS (125) d'un paiement par le sujet pour le test de laboratoire.

10. Procédé selon l'une quelconque des revendications 1 à 9, dans lequel le LACS (125) ne mémorise aucune information se rapportant à l'identité du sujet pendant le cours du procédé.

11. Système destiné à la fourniture à un sujet des résultats de test de laboratoire (110), comprenant :
A) un système informatique associé à un laboratoire (LACS) (125), dans lequel le LACS est configuré pour : i) recevoir une demande provenant d'un sujet (110) pour un test de laboratoire, dans lequel la demande est associée à aucun ou pas plus de trois éléments d'informations se rapportant à l'identité du sujet et dans lequel le test de laboratoire doit être réalisé avec un échantillon biologique provenant du sujet (110) ; ii) fournir à un sujet (110) un code d'identification en réponse à la demande, dans lequel le code d'identification comprend ou est lié aux informations concernant le test de laboratoire à réaliser avec l'échantillon du sujet (110) ; et iii) fournir au sujet les résultats provenant du test de laboratoire (110), après la réalisation du test de laboratoire et à la réception par le LACS (125) du code d'identification ou d'un code associé du sujet (110) ;
un récipient (130) destiné au prélèvement d'échantillon, dans lequel ledit récipient (130) présente un identifiant présentant le code d'identification ou un code associé déjà sur ledit récipient lorsque ledit récipient est reçu par le sujet (110) et avant que l'échantillon ne soit ajouté audit récipient (130) ; et
B) un dispositif de traitement d'échantillon (135), dans lequel le dispositif de traitement d'échantillon (135) est en communication avec le LACS (125) et dans lequel le dispositif de traitement d'échantillon (135) est configuré pour : i) traiter l'échantillon biologique selon le test de laboratoire demandé par le sujet (110), à la réception à l'emplacement du dispositif de traitement d'échantillon (135) dudit récipient (130) contenant l'échantillon biologique provenant du sujet (110), dans lequel ledit récipient (130) est associé au code d'identification ou à un code associé et ii) transmettre des informations associées à l'échantillon au LACS (125), dans lequel le dispositif de traitement d'échantillon (135) utilise une cartouche qui contient des réactifs pour des tests multiples, de manière à ce qu'un technicien de laboratoire manipulant le récipient (130) ne sache pas quel sous-ensemble de tests a été réellement réalisé.

12. Système selon la revendication 11, dans lequel le LACS (125) et le dispositif de traitement d'échantillon (135) sont dans des emplacements séparés.
